# EUROPEAN PATENT SPECIFICATION

(11) **EP 2 023 826 B1**
(45) Date of publication and mention of the grant of the patent: **21.06.2017**
(21) Application number: 07762117.5
(22) Date of filing: 11.05.2007
(51) Int. Cl.: A61B 17/08, A61F 2/18

(54) **MIDDLE TURBINATE MEDIALIZER**
MEDIALISATOR FÜR DIE MITTLERE NASENMUSCHEL
DISPOSITIF DE MÉDIALISATION DU CORNET NASAL MOYEN

(30) Priority: 12.05.2006 US 800176 P
(43) Date of publication of application: 18.02.2009
(73) Proprietor: ArthroCare Corporation, Austin, TX 78735 (US)
(72) Inventor: GONZALES, Donald Albert, San Antonio, TX 78249 (US); LARSON, Michael Charles, Colorado Springs, CO 80921 (US); DINGER, Fred B, III, San Antonio, TX 78230 (US); NIEDERAUER, Gabriele G., San Antonio, TX 78249 (US); WRANA, Jeffrey S., San Antonio, TX 78240 (US)
(74) Representative: Leach, Sean Adam
(86) International application number: PCT/US2007/068743
(87) International publication number: WO 2007/134215

(56) References cited:
- EP-A- 1 297 788
- WO-A-03/008003
- US-A- 5 094 233
- US-A1- 2002 077 661
- US-A1- 2005 113 850
- US-A1- 2005 113 850

## Description

### Background of the Invention

Sinusitis is a progression of inflammation, stasis, infection, and continued inflammation. Typically, the beginning of all sinus infections is either allergy or viral infection. Both of these conditions lead to swelling of the sinus and nasal mucosa that when severe enough, causes the small holes, called ostia, of the sinuses to close. Once the ostia is closed, the environment inside the sinuses, specifically the maxillary sinus, becomes conducive to bacterial growth. The way this typically occurs is that once the ostia is shut, the oxygen content of the sinus drops and the fluid inside the sinus is unable to escape which leads to further inflammation. The reduced oxygen content and inflammation disrupts the ability of the cilia of the cells of the sinus to operate properly which leads to further stasis.

The typical patient that is seen by the otolaryngologist is started on antibiotics. Usually the antibiotic course can be as long as six weeks to eradicate the bacteria and bring the sinuses back to normal. For those patients in whom antibiotics do no relieve the problem, the only alternative is surgery. Although sinus and nasal surgeries are now common with 500,000 to 700,000 of such surgeries being performed annually in the U.S., these surgeries are typically both destructive and permanent. Around 10% of patients who undergo sinus surgery have scarring that leads to continued sinus problems which frequently require revision surgery.

One frequent problem is postoperative adhesions. These adhesions occur between the middle turbinate and the adjacent nasal areas. One particular problem is the adhesion of the middle turbinate to the lateral nasal wall. Some surgeons have proposed removing the lower half of the middle turbinate to avoid this problem. This procedure, however, has its own problems (*e.g.*, crust formation, nasal hygiene issues).

Other solutions that have been suggested include placing a suture through the middle turbinate on one side of the nose, through the nasal septum, and then through the middle turbinate on the other side before the suture is tied off. Such a suture draws the middle turbinates medially and prevents the formation of adhesions between the middle turbinate and the lateral nasal wall. However, this suture is difficult and time-consuming to place and requires the puncturing of three separate structures in the nose. This can lead to discomfort for the patient, bleeding, infection, and other complications.

Another solution surgeons have proposed is the use of various packing materials and splints. The use of these materials and devices however leads to the formation of scar tissue, which is undesirable and can lead to airway obstruction and infection. The adhesion of the middle turbinate to adjacent structures in the nose remains a problem in nasal and sinus surgery.

US 2005/0113850 discloses a medical instrument for use in the nasal cavities and suitable for placing a medical device for attachment of the middle turbinate to the nasal septum.

US 2002/0077661 discloses multi-barbed wound closure devices and methods for establishing and maintaining two sides of a wound in apposition.

EP 1 297 788 discloses an absorbable bone anchor that can toggle in two planes for secure anchorage within a bone cavity.

WO 03/008003 discloses an adhesive for sealing a wound, the adhesive comprising a cyanoacrylate.

None of these applications disclose an instrument for the insertion of a medical device, wherein the medical device is configured to be placed between a middle turbinate and a nasal septum, and wherein the instrument comprises means for applying pressure to the middle turbinate and the nasal septum around the medical device in order to attach the middle turbinate to the nasal septum by means of the medical device.

Given this serious and common complication of sinus surgery, there remains a need in the art for preventing the formation of adhesions between the middle turbinate and adjacent nasal structures, particularly the lateral nasal wall. The desired solution preferably limits or eliminates the complications of the other proposals which have been used including infection, scar tissue formation, adhesions, bleeding, and patient discomfort.

### Summary of the Invention

The invention is disclosed in claim 1 with preferred embodiments disclosed in the dependent claims.

The present invention provides a system for reducing the adhesions formed in a patient's nasal cavity following a sinus or nasal procedure. In particular, the inventive system reduces the formation of adhesions between the lateral nasal wall and the middle turbinate by attaching the middle turbinate to the nasal septum. This system pulls the middle turbinate medially to avoid the formation of adhesions which may lead to further complications after sinus or nasal surgery. The attachment of the middle turbinate to the nasal septum may be temporary or permanent. This system may also be used prior to surgery to pull the middle turbinate away from the uncinate process to make surgeries in this area easier.

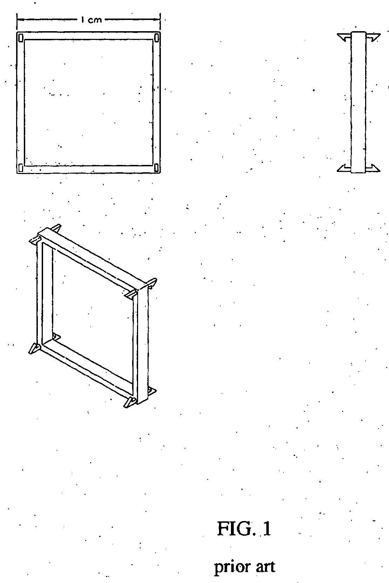

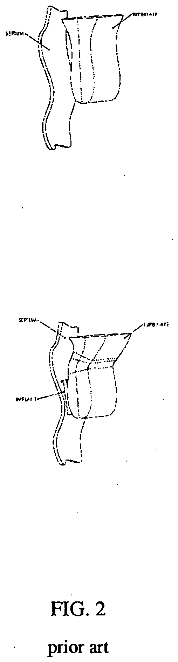

In one aspect, a medical device for medializing the middle turbinate. Suitable to be used with the instrument for inserting a medical device according to claim 1 is provided. As shown in *Figures 1* and *2*, in certain embodiments, the device is a wafer with a means for attaching the wafer to a surface (*e.g.*, a mucosal surface) on both sides of the wafer. The means for attaching may include a tissue glue (*e.g.*, cyanoacrylate, fibrin sealant), hooks, barbs, pins, staples, arrows, *etc.* The wafer thereby can bring two structures together. The device is particularly useful in attaching the middle turbinate to the nasal septum thereby preventing the formation of adhesions between the middle turbinate and the lateral nasal wall which can lead to complications after nasal and sinus surgeries. The wafer can be any shape including discs, rings, triangular-shaped wafers, polygonal-shaped wafers, zig-zag, *etc*. In certain instances, the wafer may include contours to fit comfortably inside the nose of the patient. For example, the wafer may include a contour for the middle turbinate on one side and be flat on the side that abuts the nasal septum. The wafer is typically approximately 1 cm by approximately 1 cm so that it can rest comfortably inside the nose of the patient between the middle turbinate and nasal septum. The device is approximately 0.75 mm or less in thickness. The wafer may be made from any biocompatible material.

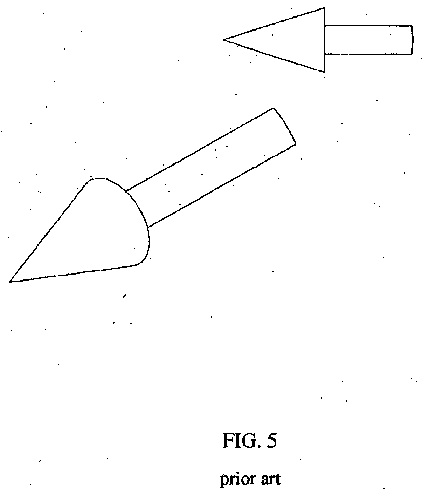

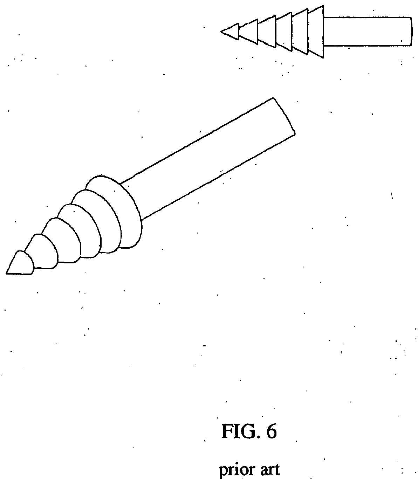

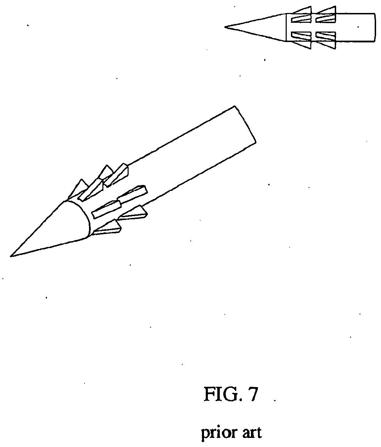

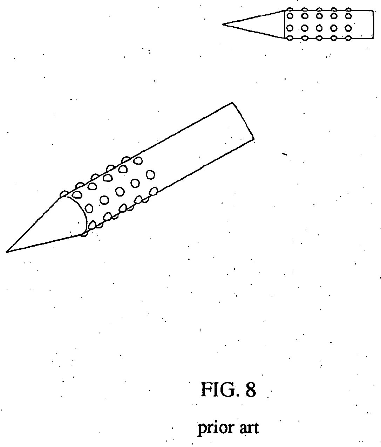

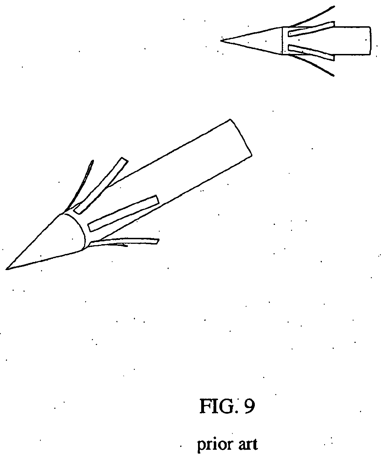

In another embodiment, the device comprises a sling-like portion to securely grasp the turbinate and barbs, adhesives, or other fixation means for attaching the device with the turbinate to the nasal wall. In yet another embodiment, the device is an arrow-like device or pin used to fix the middle turbinate to the nasal wall by pinning the turbinate. *See*, *e.g.*, *Figures 5-9**.* Such devices or pins may have protrusions, flanges, barbs, coatings, or bumps on their surfaces to prevent the device from falling out. *See*, *e.g.*, *Figures 7-9**.*

Preferably, the wafer or other device is made from a bioabsorbable material, for example, a PLGA co-polymer. Therefore, after the patient's nose has healed, the wafer or other device is absorbed by the body, thus avoiding the permanent attachment of the middle turbinate to the nasal septum. In certain embodiments, the wafer or other device is made of a non-bioresorbable material; thus, the device, if needed, can be removed later or left in place permanently.

The invention is suitable for use in a method for medializing the middle turbinate. In certain embodiments, the wafer as described above is inserted into the nose of patient between the middle turbinate and the nasal septum, and pressure is applied to the middle turbinate and nasal septum to attach these two structures via the wafer. In another embodiment, tissue adhesive (*e.g*., a cyanoacrylate adhesive) rather than the inventive wafer is used to adhere the middle turbinate to the nasal septum. In still another embodiment, the wafer may be used in conjunction with a tissue adhesive. In still other embodiments, the middle turbinate is pinned to the nasal septum. In yet other embodiments, the sling-like device is used to draw the middle turbinate toward the nasal septum. By any of these approaches, the middle turbinate is adhered to the nasal septum thereby moving the middle turbinate medially. The method is typically performed during a nasal or sinus procedure or surgery (*e.g.*, endoscopic sinus surgery). The device may be implanted at the beginning of a procedure to pull the middle turbinate away from the uncinate process to make the procedure easier. This may move the middle turbinate out of the way for better visualization of the lateral wall and such structures as the ostia leading to the paranasal sinuses and the uncinate process. The device may then be left in place to prevent the formation of adhesions between the middle turbinate and the nasal septum. The wafer or other device may be implanted using medical devices for endoscopic surgery or may be implanted using specially designed tools for using the device. After the device is implanted or adhesive is applied, it typically stays in place long enough for the mucosa of the nasal passage to heal. The device or adhesive may stay in place for a time ranging from 1 week to 6 months. Once the mucosa has healed and there is no longer a risk of adhesions forming, the device may be removed or be absorbed by the patient's body. The device may also fall out of place, be swallowed by the patient along with mucus, and be safely degraded by the digestive system of the patient.

An exemplary method not forming past of the present invention is a method for medializing the middle turbinate using a tissue glue (*e.g*., cyanoacrylate, fibrin sealant) alone. Tissue glue is applied to the middle turbinate and/or the nasal septum, and pressure is applied to these two structures so that they come in contact for a sufficient time for the tissue glue to set. The adhesion of the middle turbinate to the nasal septum allows for the healing of the nasal mucosa without the risk of adhesions developing between the middle turbinate and the lateral nasal wall. Over time, the tissue glue breaks down, and the middle turbinate is subsequently released from the nasal septum. In the case of using a tissue glue such as cyanoacrylate alone, the glue may need to be reapplied by the treating physician every week or as needed until the mucosa heals and there is limited risk of adhesions forming.

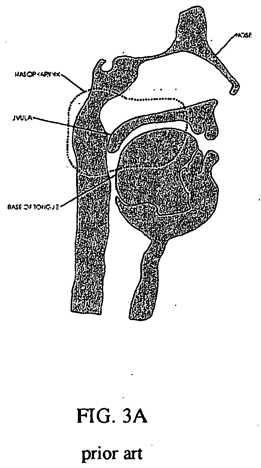

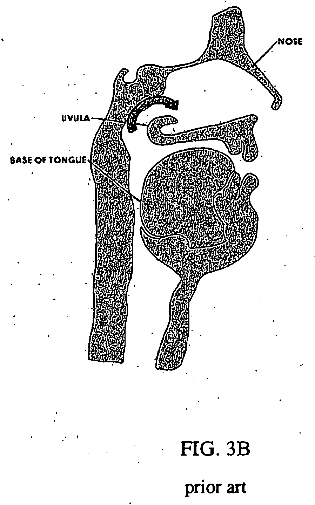

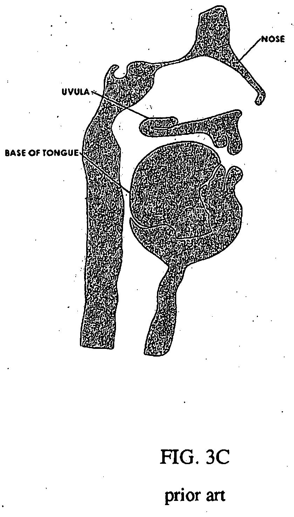

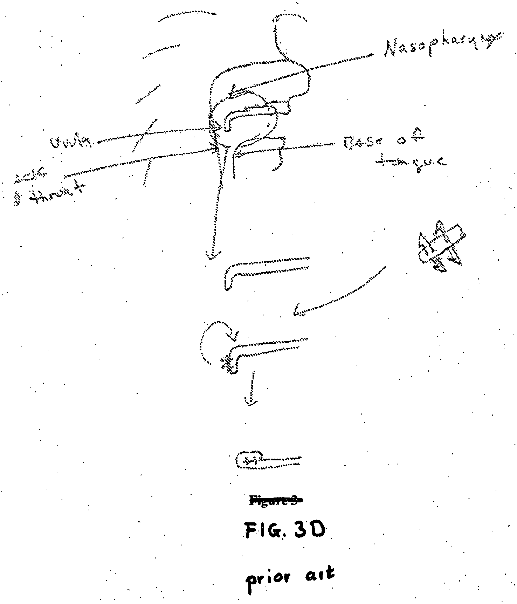

The invention is suitable for use in an exemplary method of using the medical device or tissue adhesive to attach the uvula to the nasopharyngeal side of the soft palate. Such a procedure is illustrated in *Figures 3*A-D. The procedure is particularly useful in treating snoring or sleep apnea. The attachment may be permanent or temporary as needed.

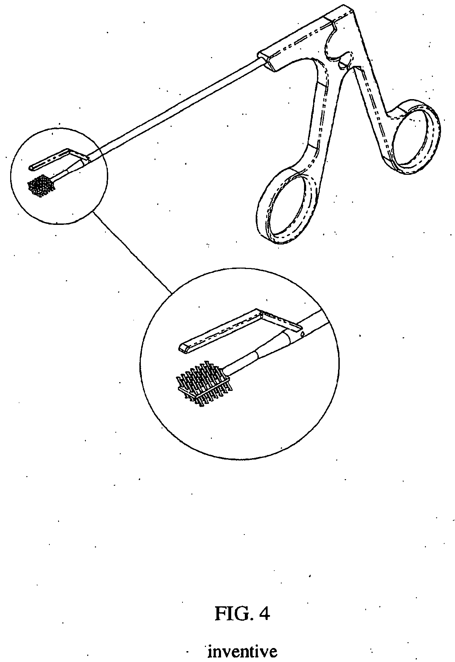

The invention provides an instrument for inserting the medical device into the nose of a patient. The instrument includes a comfortable grip and an elongated end with a means for holding and releasing the medical device in place. The invention provides an instrument for applying pressure to the middle turbinate and nasal septum around the medical device in order to attach the middle turbinate to the nasal septum by means of the medical device. An example of an instrument for inserting the wafer is shown in *Figure 4*.

In another aspect, the invention provides a kit including the instrument for inserting a medical device according to claim 1 and the medical device. The kit may also include tissue glue *(e.g.* cyanoacrylate, fibrin sealant), pharmaceutical agents (*e.g.*, steroids, non-steroidal anti-inflammatory agents, antibiotics), an instrument for removing the inventive device, instructions for inserting the inventive medical device, *etc.* Typically, these items are conveniently packaged for the use by a treating physician. In certain embodiments, the items are sterilely packaged.

The present invention fills a need in nasal and sinus surgery for preventing adhesions after surgery by temporarily adhering the middle turbinate to the nasal septum. After the nasal mucosa has healed sufficiently the attachment naturally breaks down or is manually removed, thereby restoring the natural anatomy of the nasal passage. The inventive system reduces the complications following sinus and nasal surgery.

### Brief Description of the Drawing

*Figure 1* shows an example of the wafer-like medical device suitable to be used with the instrument for inserting a medical device according to claim 1 with barbs for attaching to the nasal mucosa of the septum and the mucosa of the middle turbinate.
*Figure 2* shows the placement of the device suitable to be used with the instrument for inserting a medical device according to claim 1 and the resulting medialization of the middle turbinate.
*Figure*s *3*A-D show the use of a medical device suitable to be used with the instrument for inserting a medical device according to claim 1 with barbs to attach the uvula to the nasopharyngeal side of the soft palate.
*Figure 4* is an illustration of the inventive instrument for placing the wafer for attaching the middle turbinate to the nasal septum.
*Figure 5* shows exemplary pins suitable to be used with the instrument for inserting a medical device according to claim 1 for attaching the nasal mucosa of the septum and the mucosa of the middle turbinate.
*Figure 6* shows another design of the pins suitable to be used with the instrument for inserting a medical device according to claim 1 that have ridges on the pointed tip.
*Figure 7* shows another design of the pins suitable to be used with the instrument for inserting a medical device according to claim 1 with protrusions for preventing the pin from dislodging.
*Figure 8* shows another design of the pins with bump-like protrusions.
*Figure 9* shows another design of the pins with barbs.

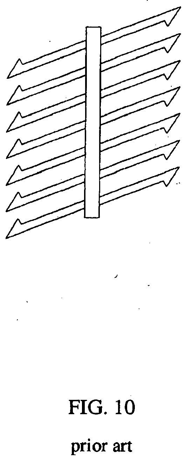

*Figure 10* shows a wafer suitable to be used with the instrument for inserting a medical device according to claim 1 with barbs for attaching the nasal mucosa of the septum to the mucosa of the middle turbinate.

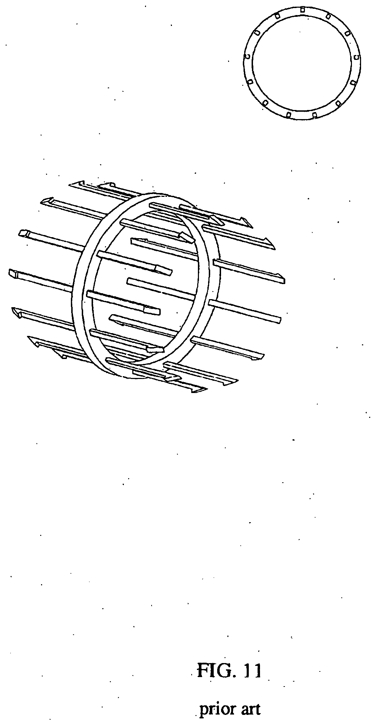

*Figure 11* shows a circular design with barbs suitable to be used with the instrument for inserting a medical device according to claim 1. for attaching the nasal mucosa of the septum to the mucosa of the middle turbinate.

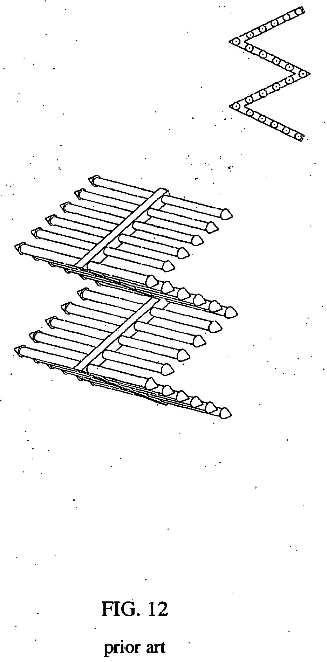

*Figure 12* shows a zig-zag design of the medical device suitable to be used with the instrument for inserting a medical device according to claim 1.

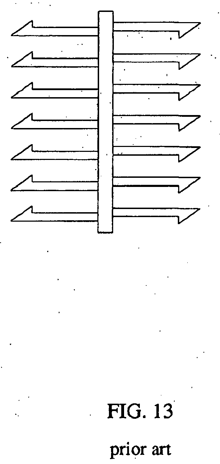

*Figure 13* shows a side view of an exemplary medical device suitable to be used with the instrument for inserting a medical device according to claim 1.

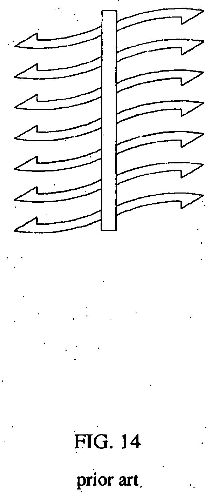

*Figure 14* shows a side view of another exemplary medical device suitable to be used with the instrument for inserting a medical device according to claim 1. with curved barbs.

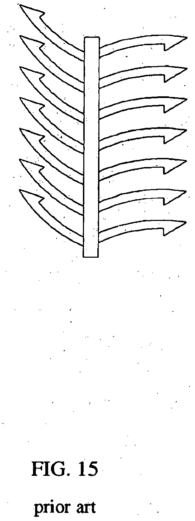

*Figure 15* shows a side view of another exemplary medical device suitable to be used with the instrument for inserting a medical device according to claim 1. with curved barbs.

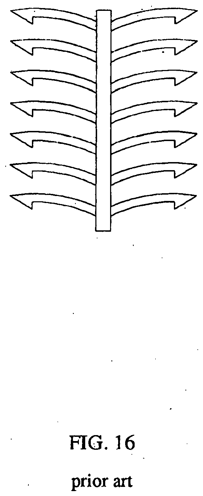

*Figure 16* shows a side view of another exemplary medical device suitable to be used with the instrument for inserting a medical device according to claim 1. with curved barbs.

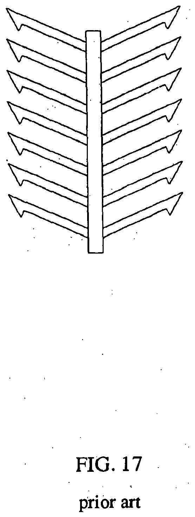

*Figure 17* shows a side view of another exemplary medical device suitable to be used with the instrument for inserting a medical device according to claim 1. with slanted barbs with respect to the surface of the wafer.
*Figure 18* shows another design for the medical device suitable to be used with the instrument for inserting a medical device according to claim 1 with two barbs for attachment.
*Figure 19* shows a design with four barbs suitable to be used with the instrument for inserting a medical device according to claim 1..
*Figure 20* shows a planar design for the medical device suitable to be used with the instrument for inserting a medical device according to claim 1.
*Figures 21*A,B show suitable to be used with the instrument for inserting a medical device according to claim 1 a sling-type device suitable to be used with the instrument for inserting a medical divice according to claim 1 in which the sling portion is slipped around the turbinate and then the device, is secured to the nasal wall with piercing arrows or barbs.

### Detailed Description of the invention

The present invention provides a system for medializing the middle turbinate following and/or during nasal or sinus surgery. The invention stems from the recognition that attaching the middle turbinate to the nasal septum, thereby drawing the middle turbinate medially would prevent the formation of adhesions between the middle turbinate and lateral wall. These adhesions are known to cause further complications post surgery including paranasal sinus blockage. The inventive system prevents the formation of adhesions between the middle turbinate and the lateral nasal wall and therefore the subsequent complication. These adhesions frequently require post-revision surgery to remove the adhesions. The invention not only provides an instrument for inserting a medical device for use in medializing the middle turbinate but also provides kits, instruments for placing and removing the inventive devices, and the invention is suitable for procedures for medializing the middle turbinate.

A patient suffering from nasal or sinus disease (*e.g.*, allergies, infection) having undergone a sinus or nasal procedure is at a substantial risk of developing adhesions between various structures in the nasal passage due to trauma to the mucosal surfaces. In order to prevent the formation of adhesions, particularly between the lateral nasal wall and the middle turbinate, the middle turbinate is attached at least temporarily to the nasal septum. In certain embodiments, the middle turbinate is attached to the nasal septum prior to starting the procedure or surgery in order to make the surgery easier. The attachment can then be left in place after the procedure or surgery is concluded. This attachment is accomplished using a medical device suitable to be used with the instrument for inserting a medical device according to claim 1 such as a wafer or pin with means for attaching middle turbinate to the nasal septum or in examples not forming past of the invention by using a tissue glue such as a cyanoacrylate adhesive, fibrin sealant, or other natural or synthetic adhesive. In most instances, the attachment is temporary. Typically, the attachment is only in place for the length of time needed for the nasal mucosa to heal. Once the mucosa is healed, the chance of adhesions forming is greatly reduced. The attachment may be manually severed, or the means for attaching the middle turbinate and the nasal septum may degrade over time. For example, the device may be absorbed by the patient's body. The device may fall out of place and be harmlessly swallowed by the patient and degraded in the patient's digestive system. Or the adhesive may break down releasing the middle turbinate from the nasal septum.

The attachment whether by medical device suitable to be used with the instrument for inserting a medical device according to claim 1 or exemplary adhesive alone may last from week to 24 months depending on the judgment of the treating physician. In certain embodiments, the attachment lasts from 2 weeks to 8 weeks, or 3 weeks to 6 weeks. In other embodiments, the attachment lasts for approximately 1 month, 2 months, 3 months, 4 months, 5 months, or 6 months. In other embodiments, the attachments last for approximately. 9 months, 12 months, 18 months, or 24 months. If longer attachment is necessary, the procedure may be repeated once, twice, three times, or more depending upon the patient and the judgment of the treating physician. In certain examples where a tissue adhesive alone is used, the adhesive may need to be reapplied every few days, every week, every two weeks, or as needed until the nasal mucosa is healed. In certain examples where a cyanoacrylate adhesive is used, the adhesive is reapplied approximately every week.

As described above for drawing medially the middle turbinate, the device suitable to be used with the instrument for inserting a medical device according to claim 1 may also be used to attach the uvula to the nasopharyngeal side of the soft palate. Such an attachment is particularly useful in patients who snore or patients who suffer from sleep apnea. The attachment may also be used to move the uvula out of the way for a procedure involving the oronasopharynx. The attachment may be temporary or permanent. The wafer or other medical device as described herein is inserted into the oronasopharynx of the patient either through the nose or mouth. The device is then used to attach the soft palate to the uvula. Pressure may be applied to the uvula and soft palate to attach these two structures via the device. In one particular example, tissue adhesive (*e.g.*, a cyanoacrylate adhesive) rather than a device is used to adhere the uvula to the nasal septum. In still another embodiment, a device may be used in conjunction with a tissue adhesive. The method is typically performed during a procedure or surgery. The device may be implanted using medical devices for endoscopic surgery or may be implanted using specially designed tools for using the device.

As will be appreciated by those of skill in the art, the inventive system may be used in attaching other structures in the body to each other (*e.g.*, in the oronasopharynx, gastrointestinal system, genitourinary system, *etc*.). In certain examples the system is used in the oronasopharynx and attached to one or more of the following structures: turbinate, nasal septum, uvula, hard palate, soft palate, tonsils, tongue, gingiva, epiglottis, walls of the sinus, and sides of the oral cavity. The inventive system is particularly useful in attaching mucosal surfaces. In certain examples, inventive system is not used to approximate wound surfaces. In other examples, the inventive system is used to approximate wound surfaces.

In one embodiment, the medical device suitable to be used with the instrument for inserting a medical device according to claim 1 is a thin wafer with both sides of the wafer haying means for attaching the wafer to a surface. Therefore, the wafer can be used to bring two structures such as the middle turbinate and the nasal septum together. The wafer can be any shape or size capable of being placed into the space between the middle turbinate and nasal septum of a patient, preferably a human patient. In certain embodiments, the wafer is circular. In other embodiments, the wafer is triangular shaper, rectangular shaped, or polygonal shaped. In yet other embodiments, the wafer is a ring. In certain embodiments, the wafer is a zig-zag shape. The surface area of the sides of the wafer should provide a large enough surface area to adequately attach to the middle turbinate and nasal septum so that the middle turbinate can be pulled medially. The wafer is typically approximately 0.2 cm-2 cm in length by approximately 0.2 cm-2 cm in width. In certain embodiments, the length ranges from approximately 0.5 cm to approximately 1.5 cm. In certain embodiments, the length ranges from approximately I cm to approximately 2 cm. In certain embodiments, the length ranges from approximately 1.5 cm to approximately 2 cm. In certain embodiments, the length ranges from approximately 0.25 cm to approximately 0.75 cm. In certain embodiments, the length ranges from approximately 0.5 cm to approximately 1 cm. In certain embodiments, the width ranges from approximately 0.5 cm to approximately 1.5 cm. In certain embodiments, the width ranges from approximately 1 cm to approximately 2 cm. In certain embodiments, the width ranges from approximately 1.5 cm to approximately 2 cm. In certain embodiments, the width ranges from approximately 0.25 cm to approximately 0.75 cm. In certain embodiments, the width ranges from approximately 0.5 cm to approximately 1 cm. In certain embodiments, the wafer is approximately 1.5 cm by approximatey 1.5 cm. In certain embodiments, the wafer is approximately 1 cm by approximatey 1 cm. In certain embodiments, the wafer is approximately 0.75 cm by approximatey 0.75 cm. In certain embodiments, the wafer is approximately 0.5 cm by approximatey 0.5 cm. In certain embodiments, the wafer is approximately 0.25 cm by approximatey 0.25 cm. For pediatric patients, the wafer may be smaller, that is, less than 1 cm by 1 cm. Also, the wafer may be smaller where more than one wafer is being used to attach the middle turbinate to the nasal septum. The wafer is approximately 0.75 mm in thickness; however, the thickness of the wafer may vary from less than 0.2 mm to approximately 0.5 cm. In certain embodiments, the thickness of the wafer is in the range of approximately 0.5 mm to approximately 1.5 mm. In other embodiments, the wafer is a thin film of less than 0.2 mm in thickness.

The means on the wafer or other device suitable to be used with the instrument for inserting a medical device according to claim 1 described herein for attaching the device to a surface such as the surface of the middle turbinate or the surface of the nasal septum include mechanical means of forming an attachment or, in examples not forming part of the claimed invention, may include any chemical adhesive. The means for attaching is preferably suitable for attaching the device to a mucosal surface. In certain examples when a chemical adhesive is used, the adhesive is a cyanoacrylate adhesive. In other examples, a similar synthetic glue is used as the adhesive. In other examples when an adhesive is used, the adhesive is a fibrin sealant or other natural substance such as mussel adhesive protein, frog glue, *etc..* These adhesives have been shown useful in closing wounds and are commercially available. The adhesive may be applied to the device immediately before implanting the device in the patient. Mechanical means for forming an attachment include pins, staples, rivets, barbs, or hooks on the surface of the device which allow attachment to a surface. The surface of the wafer or other device may also be constructed to have a fibrous surface similar to Velcro^{®} for attaching the device to a tissue such as one with a mucosal surface. These attachment means typically extend less that approximately 1 cm from the surface of the wafer or other device, more preferably, less than 0.5 cm from the surface of the device. In certain embodiments, they extend less than 1 mm from the surface. Usually multiple pins, staples, rivets, barbs, or hooks are used to provide a secure attachment. These means typically do not puncture through the entire nasal structure. In certain embodiments, the mechanical means only penetrate the mucosa. In certain embodiments, an adhesive (e.g., cyanoacrylate, fibrin sealant, mussel adhesive protein, frog glue) is used in conjunction with a mechanical means for attachment.

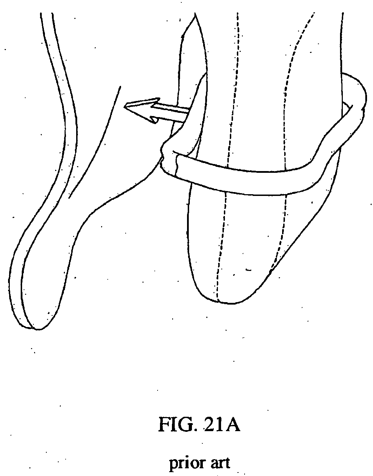

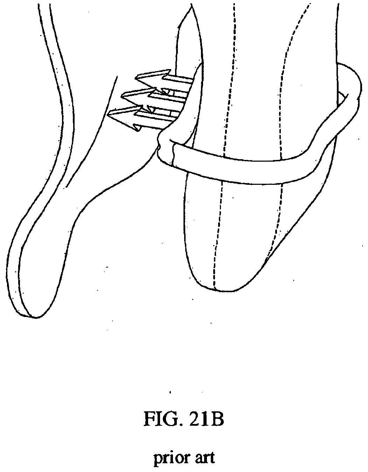

In another example, the device suitable to be used with the instrument for inserting a medical device according to claim 1 comprises a sling-like or pouch-like portion that is slipped around the middle turbinate and barbs or arrows for securing the device to the nasal septum. The device thereby draws the middle turbinate medially toward the nasal septum. The sling portion may be made of a thin suture-like material, or it may be made of a wider material, which is solid or mesh-like. An illustration of such a device is shown in *Figures 21*A,B.

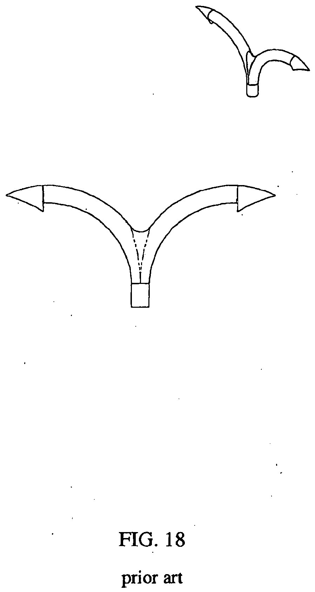

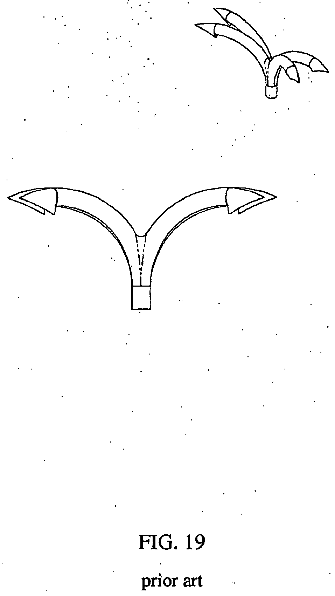

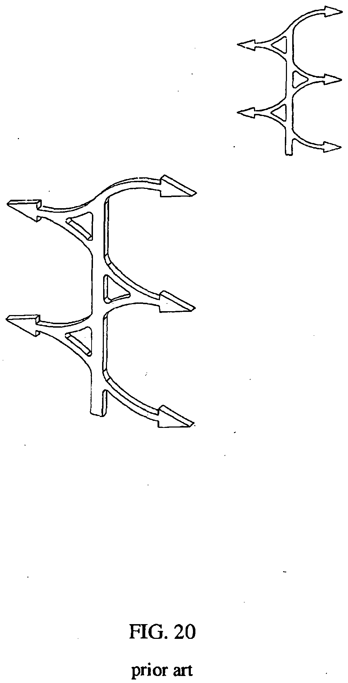

In yet another embodiment, the device suitable to be used with the instrument for inserting a medical device according to claim 1 is a pin for attaching the middle turbinate to the nasal septum. These devices are typically less than 2 cm in length. In certain embodiments, the devices are approximately 0.5 cm to 1.5 cm in length. In certain embodiments, the devices are approximately 0.25 cm, approximately 0.5 cm, approximately 0.75 cm, approximately 1 cm, approximately 1.25 cm, approximately 1.5 cm, approximately 1.75 cm, or approximately 2 cm in length. The surface of the pin may include protrusions to prevent the pin from coming out. The protrusions may be small barbs, bumps, ridges, *etc*. The pin may also be coated to prevent the pin for easily dislodging. The pin may also be coated to make it more biocompatible or allow for release of a bioactive agent. Exemplary designs for such pin devices are shown in *Figures 5-9**.* Other devices with two or more pins are also included within the invention as shown in *Figures 18-20**.* Such devices may be smaller than the wafer devices.

Any of the devices suitable to be used with the instrument for inserting a medical device according to claim 1 can be made of any biocompatible material. Preferably, the device is made of a biodegradable material. In certain embodiments, the material is a biodegradable polymer. The material may be synthetic (*e.g.*, polyesters, polyanhydrides) or natural (*e.g*., proteins, rubber, polysaccharides). Preferably, the device is made of a biodegradable material. In certain embodiments, the material is a biodegradable polymer. In certain embodiments, the material is a homopolymer. In certain embodiments, the material is a co-polymer. In other embodiments, the material is a block polymer. In other embodiments, the material is a branched polymer. In other embodiments, the material is a cross-linked polymer. In certain embodiments, the polymer is a polyester, polyurethane, polyvinyl chloride, polyalkylene (*e.g.*, polyethylene), polyolefin, polyanhydride, polyamide, polycarbonate, polycarbamate, polyacrylate, polymethacrylate, polystyrene, polyurea, polyether, polyphosphazene, poly(ortho esters), polycarbonate, polyfumarate, polyarylate, polystyrene, or polyamine. In certain embodiments, the polymers is polylactide, polyglycolide, polycaprolactone, polydioxanone, polytrimethylene carbonate, and copolymers thereof. Polymers that have been used in producing biodegradable implants and are useful in preparing the inventive devices include alpha-polyhydroxy acids; polyglycolide (PGA); copolymers of polyglycolide such as glycolide/L-lactide copolymers (PGA/PLLA), glycolide/D,L-lactide copolymers (PGA/PDLLA), and glycolide/trimethylene carbonate copolymers (PGA/TMC); polylactides (PLA); stereocopolymers of PLA such as poly-L-lactide (PLLA), poly-D,L-lactide (PDLLA), L-lactide/D,L-lactide copolymers; copolymers of PLA such as lactide/tetramethylglycolide copolymers, lactide/trimethylene carbonate copolymers, lactide/δ-valerolactone copolymers, lactide ε-caprolactone copolymers, polydepsipeptides, PLA/polyethylene oxide copolymers, unsymmetrically 3,6-substituted poly-1,4-dioxane-2,5-diones; polyhydroxyalkanate polymers including poly-beta-hydroxybutyrate (PHBA), PHBA/beta-hydroxyvalerate copolymers (PHBA/HVA), and poly-beta-hydroxypropionate (PHPA); poly-p-dioxanone (PDS); poly-δ-valerolatone; poly-ε-caprolactone; methylmethacrylate-N-vinyl pyrrolidone copolymers; polyesteramides; polyesters of oxalic acid; polydihydropyrans; polyalkyl-2-cyanoacrylates; polyurethanes (PU); polyvinyl alcohol (PVA); polypeptides; poly-beta-maleic acid (PMLA); poly(trimethylene carbonate); poly(ethylene oxide) (PEO); poly(β-hydroxyvalerate) (PHVA); poly(ortho esters); tyrosine-derived polycarbonates; and poly-beta-alkanoic acids. In certain embodiments, the polymer is a polyester such as poly(glycolide-co-lactide) (PLGA), poly(lactide), poly(glycolide), poly(D,L-lactide-co-glycolide), poly(L-lactide-co-glycolide), poly-(β-hydroxybutyrate, and polyacrylic acid ester. In certain embodiments, the device is made of PLGA. In certain embodiments, the device is made of 85% D,L-lactide and 15% glycolide co-polymer. In certain embodiments, the device is made of 50% D,L-lactide and 50% glycolide co-polymer. In certain embodiments, the device is made of 65% D,L-lactide and 35% glycolide co-polymer. In certain embodiments, the device is made of 75% D,L-lactide and 25% glycolide co-polymer. In certain embodiments, the device is made of 85% L-lactide and 15% glycolide co-polymer. In certain embodiments, the device is made of 50% L-lactide and 50% glycolide co-polymer. In certain embodiments, the device is made of 65% L-lactide and 35% glycolide co-polymer. In certain embodiments, the device is made of 75% L-lactide and 25% glycolide co-polymer. In certain embodiments, the device is made of poly(caprolactone). In certain embodiments, the device is made of Pebax, Polyimide, Braided Polyimide, Nylon, PVC, Hytrel, HDPE, or PEEK. In certain embodiments, the device is made of a fluoropolymer such as PTFE, PFA, FEP, and EPTFE. In certain embodiments, the device is made of latex. In other embodiments, the device is made of silicone. The polymer typically has a molecular weight sufficient to be shaped by molding or extrusion. The device is typically made of a material that is bioabsorbed after the device is not longer needed. For example, the device may degrade after I week, 2 weeks, 3 weeks, 1 month, 2 months, 3 months, 4 months, 5 months, 6 months, 9 months, 1 year, 1.5 years, 2 years, 3 years, *etc.* The polymer used to make the device may be selected based on its degradation profile. As would be appreciated by one of skill in this art, the composition of the wafer may be varied to achieve the desired lifetime *in vivo* of the wafer.

In other embodiments, the device suitable to be used with the instrument for inserting a medical device according to claim 1 is made of a metal. In other embodiments, the device is made of an alloy. In certain embodiments, the device is made of stainless steel. In certain embodiments, the device is made of a magnesium alloy (*e.g*., magnesium based alloy AE21). In certain embodiments, the device is made of titanium. In certain embodiments, the device is made of a titanium alloy. In certain embodiments, the device is made of a superelastic alloy such as Nitinol. Metal devices may be optionally coated with a biocompatible coating. In the case where the device is made of a metal, the device may be inserted permanently or may be removed manually after the device is no longer needed.

The device suitable to be used with the instrument for inserting a medical device according to claim 1. Suitable to be used with the instrument for may be coated with a biocompatible material. In certain embodiments, the device is made of or is coated with a timed-release formulation of a pharmaceutical agent. For example, a steroid, analgesic, anti-inflammatory agent, or antibiotic may be released by the wafer. In certain embodiments, the device is coated with a bioactive agent. Bioactive agents include small molecules, drugs, polynucleotide, proteins, peptides, *etc.* In certain embodiments, the bioactive agent may promote wound healing. In certain embodiments, the bioactive agent stimulates the formation of a desired tissue. In certain embodiments, the bioactive agent accelerates the integration of the turbinate with the nasal septum. In yet other embodiments, the tube may be coated with a material to prevent cell growth such as a cytotoxic agent. The device may also be coated with a substance to prevent the formation of adhesions. For example, the device may be coated with a polysaccharide such as hyaluronate. The device may also be coated with a polymeric coating such as Teflon.

The medical device and the inventive instrument for inserting a medical device according to claim 1 may be packaged in kits for convenience. In certain embodiments, the kits may also include all or some of the following items: an instrument for removing the device, adhesive, pharmaceutical agents, nasal sprays, gauze, bandages, disinfectant, and instructions for using the device. In certain embodiments, the kits are sterilely package for convenient use by a surgeon or other medical professional.

## Claims

1. An instrument for implanting a medical device, wherein:
the medical device is configured to be placed between a middle turbinate and a nasal septum and configured to approximate a mucosal surface of the middle turbinate to a mucosal surface of the nasal septum, the device comprising a body comprising two opposed sides, and at least one hook, pin, arrow or barb on each side, wherein during use, the at least one hook, pin, arrow or barb on one side attaches the device to the mucosal surface of the middle turbinate and the at least one hook, pin, arrow or barb on the opposing side attaches the device to the mucosal surface of the nasal septum, thereby approximating the two mucosal surfaces; and wherein the instrument includes a comfortable grip and an elongated end with a means for holding and releasing the medical device in place and means for applying pressure to the middle turbinate and the nasal septum around the medical device in order to attach the middle turbinate to the nasal septum by means of the medical device.

2. The instrument of claim 1, wherein the device is made of a bioabsorbable material.

3. The instrument of claim 2, wherein the two mucosal surfaces are temporarily approximated and the device is bioabsorbed after the two mucosal surfaces are no longer approximated.

4. The instrument of claim 2, wherein the bioabsorbable material comprises a polyester, a polyanhydride, a polyamide, a polycarbonate, a polycarbamate, a polyacrylate, a polymethacrylate, a polystyrene, a polyurea, a polyether, or a polyamine, in particular a polyester.

5. The instrument of any of claims 1 to 4, wherein the device is made of PLGA, in particular wherein the device is made of a 85% D,L-lactide and 15% glycolide co-polymer; a 75% D,L-lactide and 25% glycolide co-polymer; a 65% D,L-lactide and 35% glycolide co-polymer; or a 50% D,L-lactide and 50% glycolide co-polymer.

6. The instrument of any of claims 1 to 5, wherein the device is approximately 0.2-2 cm in length and approximately 0.2-2 cm in width, in particular wherein the device is approximately 1 cm by approximately 1 cm.

7. The instrument of any of claims 1 to 6, wherein the body of the device is less than approximately 2 mm thick and preferably is approximately 0.75 mm thick.

8. The instrument of any of claims 1 to 7, wherein the device is coated with a bioactive agent.

9. The instrument of claim 8, wherein the bioactive agent comprises a drug, a polynucleotide, a protein or a peptide.

10. The instrument of claim 8, wherein the bioactive agent promotes wound healing.

11. The instrument of any of claims 1 to 7, wherein the device is made of or is coated with a timed-release formulation of a pharmaceutical agent.

12. The instrument of claim 11, wherein the pharmaceutical agent comprises a steroid, analgesic, anti-inflammatory agent, or an antibiotic.

13. A kit comprising the instrument for inserting the medical device and the medical device of any of claims 1 to 12 and optionally further comprising:
instructions for using the device.

## Patentansprüche

1. Instrument zum Implantieren einer medizinischen Vorrichtung, wobei:
die medizinische Vorrichtung zum Platzieren zwischen eine mittlere Nasenmuschel und ein Nasenseptum konfiguriert ist und zur Annäherung einer Schleimhautoberfläche der mittleren Nasenmuschel an eine Schleimhautoberfläche des Nasenseptums konfiguriert ist, wobei die Vorrichtung einen Körper umfasst, umfassend zwei gegenüberliegende Seiten und mindestens einen Haken, Stift, Pfeil oder Widerhaken auf jeder Seite, wobei während des Gebrauchs der mindestens eine Haken, Stift, Pfeil oder Widerhaken auf einer Seite die Vorrichtung an der Schleimhautoberfläche der mittleren Nasenmuschel befestigt und der mindestens eine Haken, Stift, Pfeil oder Widerhaken auf der gegenüberliegenden Seite die Vorrichtung an der Schleimhautoberfläche des Nasenseptums befestigt, wodurch die beiden Schleimhautoberflächen angenähert werden; und wobei das Instrument einen bequemen Handgriff und ein längliches Ende mit einem Mittel zum Halten und zur Freigabe der medizinischen Vorrichtung an Ort und Stelle und Mittel zum Aufbringen von Druck auf die mittlere Nasenmuschel und das Nasenseptum um die medizinische Vorrichtung herum zum Befestigen der mittleren Nasenmuschel am Nasenseptum mithilfe der medizinischen Vorrichtung einschließt.

2. Instrument nach Anspruch 1, wobei die Vorrichtung aus einem bioabsorbierbaren Material hergestellt ist.

3. Instrument nach Anspruch 2, wobei die beiden Schleimhautoberflächen vorübergehend angenähert werden und die Vorrichtung, nachdem die beiden Schleimhautoberflächen nicht mehr angenähert sind, bioabsorbiert wird.

4. Instrument nach Anspruch 2, wobei das bioabsorbierbare Material einen Polyester, ein Polyanhydrid, ein Polyamid, ein Polycarbonat, ein Polycarbamat, ein Polyacrylat, ein Polymethacrylat, ein Polystyrol, einen Polyharnstoff, einen Polyether oder ein Polyamin, insbesondere einen Polyester umfasst.

5. Instrument nach einem der Ansprüche 1 bis 4, wobei die Vorrichtung aus PLGA hergestellt ist, insbesondere wobei die Vorrichtung aus einem Copolymer von 85% D,L-Lactid und 15% Glycolid; einem Copolymer von 75% D,L-Lactid und 25% Glycolid; einem Copolymer von 65% D,L-Lactid und 35% Glycolid; oder einem Copolymer von 50% D,L-Lactid und 50% Glycolid hergestellt ist.

6. Instrument nach einem der Ansprüche 1 bis 5, wobei die Vorrichtung ca. 0,2 bis 2 cm lang und ca. 0,2 bis 2 cm breit ist, insbesondere wobei die Vorrichtung ca. 1 cm mal ca. 1 cm misst.

7. Instrument nach einem der Ansprüche 1 bis 6, wobei der Körper der Vorrichtung weniger als ca. 2 mm dick ist und bevorzugt ca. 0,75 mm dick ist.

8. Instrument nach einem der Ansprüche 1 bis 7, wobei die Vorrichtung mit einem bioaktiven Mittel beschichtet ist.

9. Instrument nach Anspruch 8, wobei das bioaktive Mittel ein Arzneimittel, ein Polynukleotid, ein Protein oder ein Peptid umfasst.

10. Instrument nach Anspruch 8, wobei das bioaktive Mittel die Wundheilung fördert.

11. Instrument nach einem der Ansprüche 1 bis 7, wobei die Vorrichtung aus einem pharmazeutischen Mittel in einer Formulierung mit protrahierter Freisetzung hergestellt ist oder damit beschichtet ist.

12. Instrument nach Anspruch 11, wobei das pharmazeutische Mittel ein Steroid, Analgetikum, entzündungshemmendes Mittel oder ein Antibiotikum umfasst.

13. Kit, umfassend das Instrument zum Einbringen der medizinischen Vorrichtung und die medizinische Vorrichtung nach einem der Ansprüche 1 bis 12 und gegebenenfalls weiter umfassend:
Gebrauchsanleitungen für die Vorrichtung.

## Revendications

1. Instrument pour l'implantation d'un dispositif médical, dans lequel :
le dispositif médical est conçu pour être placé entre un cornet moyen et un septum nasal et conçu pour rapprocher une surface de muqueuse du cornet moyen d'une surface de muqueuse du septum nasal, le dispositif comprenant un corps comprenant deux côtés opposés, et au moins un crochet, une broche, une flèche ou un picot sur chaque côté, dans lequel pendant l'utilisation, le au moins un crochet, une broche, une flèche ou un picot sur un côté attache le dispositif à la surface de muqueuse du cornet moyen et le au moins un crochet, une broche, une flèche ou un picot sur le côté opposé attache le dispositif à la surface de muqueuse du septum nasal, rapprochant ainsi les deux surfaces de muqueuse ; et dans lequel l'instrument comprend une prise confortable et une extrémité allongée avec un moyen pour tenir et relâcher le dispositif médical en place et un moyen pour appliquer de la pression sur le cornet moyen et le septum nasal autour du dispositif médical afin d'attacher le cornet moyen au septum nasal au moyen du dispositif médical.

2. Instrument de la revendication 1, dans lequel le dispositif est fait d'un matériau biorésorbable.

3. Instrument de la revendication 2, dans lequel les deux surfaces de muqueuse sont rapprochées temporairement et le dispositif est biorésorbé une fois que les deux surfaces de muqueuse ne sont plus rapprochées.

4. Instrument de la revendication 2, dans lequel le matériau biorésorbable comprend un polyester, un polyanhydride, un polyamide, un polycarbonate, un polycarbamate, un polyacrylate, un polyméthacrylate, un polystyrène, une polyurée, un polyéther ou une polyamine, en particulier un polyester.

5. Instrument de l'une quelconque des revendications 1 à 4, dans lequel le dispositif est fait de PLGA, en particulier dans lequel le dispositif est fait d'un copolymère de D,L-lactide à 85 % et de glycolide à 15 % ; un copolymère de D,L-lactide à 75 % et de glycolide à 25 % ; un copolymère de D,L-lactide à 65 % et de glycolide à 35 % ; ou un copolymère de D,L-lactide à 50 % et de glycolide à 50 % .

6. Instrument de l'une quelconque des revendications 1 à 5, dans lequel le dispositif fait de 0,2 à 2 cm environ de longueur et de 0,2 à 2 cm environ de largeur, en particulier dans lequel le dispositif fait 1 cm environ sur 1 cm environ.

7. Instrument de l'une quelconque des revendications 1 à 6, dans lequel le corps du dispositif fait moins de 2 mm environ d'épaisseur et de préférence fait moins de 0,75 mm environ d'épaisseur.

8. Instrument de l'une quelconque des revendications 1 à 7, dans lequel le dispositif est revêtu d'un agent bioactif.

9. Instrument de la revendication 8, dans lequel l'agent bioactif comprend un médicament, un polynucléotide, une protéine ou un peptide.

10. Instrument de la revendication 8, dans lequel l'agent bioactif favorise la cicatrisation des plaies.

11. Instrument de l'une quelconque des revendications 1 à 7, dans lequel le dispositif est fait ou revêtu d'une composition à libération contrôlée d'un agent pharmaceutique.

12. Instrument de la revendication 11, dans lequel l'agent pharmaceutique comprend un stéroïde, un analgésique, un agent anti-inflammatoire ou un antibiotique.

13. Trousse comprenant l'instrument pour l'insertion du dispositif médical et le dispositif médical de l'une quelconque des revendications 1 à 12 et facultativement comprenant en outre :
des instructions pour l'utilisation du dispositif.
